(19) Europäisches Patentamt
European Patent Office
Office européen des brevets

(11) EP 2 261 279 B1

(12) # EUROPEAN PATENT SPECIFICATION

(45) Date of publication and mention of the grant of the patent:
**02.09.2015 Bulletin 2015/36**

(51) Int Cl.:
***C08K 5/3492** (2006.01)* ***C07D 251/24** (2006.01)*

(21) Application number: **10165212.1**

(22) Date of filing: **08.06.2010**

(54) **Triazine derivative, ultraviolet absorber composition and resin composition stabilized with the absorber composition**

Triazinderivat, UV-Absorberzusammensetzung und eine mit dieser Zusammensetzung stabilisierte Harzzusammensetzung

Dérivé de la triazine, composition d'absorbeur d'ultraviolets et composition de résine stabilisée avec cette composition

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **09.06.2009 JP 2009138650**
**25.09.2009 JP 2009221662**

(43) Date of publication of application:
**15.12.2010 Bulletin 2010/50**

(73) Proprietor: **Fujifilm Corporation**
**Minato-ku**
**Tokyo (JP)**

(72) Inventors:
- **Amasaki, Ichiro**
  **Kanagawa (JP)**
- **Kimura, Keizo**
  **Kanagawa (JP)**
- **Furukawa, Kazushi**
  **Kanagawa (JP)**

(74) Representative: **Hoffmann Eitle**
**Patent- und Rechtsanwälte PartmbB**
**Arabellastraße 30**
**81925 München (DE)**

(56) References cited:
**EP-A2- 0 425 429** **EP-A2- 1 213 283**
**WO-A1-02/28854** **US-A1- 2007 274 933**

- **BRUNETTI H ET AL: "DIE SYNTHESE VON ASYMMETRISCH SUBSTITUIERTEN OMICRON-HYDROXYPHENYL-SIGMA-TRIAZINEN" HELVETICA CHIMICA ACTA, VERLAG HELVETICA CHIMICA ACTA, BASEL, CH LNKD- DOI:10.1002/HLCA.19720550520, vol. 55, no. 1, 1 January 1972 (1972-01-01), pages 1566-1595, XP002034121 ISSN: 0018-019X**
- **CORWIN HANSCH ET AL: "A Survey of Hammett Substituent Constants and Resonance and Field Parameters" CHEMICAL REVIEWS, ACS, WASHINGTON, DC, US LNKD- DOI: 10.1021/CR00002A004, vol. 91, 1 January 1991 (1991-01-01), pages 165-195, XP002447343 ISSN: 0009-2665**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 261 279 B1

## Description

BACKGROUND OF THE INVENTION

1. Field of the Invention

The present invention relates to a novel triazine derivative, an ultraviolet absorber, and a resin composition.

2. Background Art

Various resins have been used in combination with an ultraviolet absorber in order to have ultraviolet absorbing properties. The ultraviolet absorber used for them is sometimes an inorganic ultraviolet absorber and sometimes an organic ultraviolet absorber. Inorganic ultraviolet absorbers (refer to, for example, JP-A-5-339033, JP-A-5-345639, and JP-A-6-56466) are excellent in durability such as weather resistance and heat resistance, but an absorption wavelength is determined by the band gap of a compound so that freedom of choice is restricted. In addition, there is no inorganic ultraviolet absorber capable of absorbing a light in a long-wavelength ultraviolet right (UV-A) region around 400 nm and those absorbing a long-wavelength ultraviolet light absorb even a light of a visible region and therefore such ultraviolet absorbers cause coloring.

On the other hand, organic ultraviolet absorbers have a structure designed with a high degree of freedom so that those having a variety of absorption wavelengths can be obtained by intentionally changing their structures.

Systems using various organic ultraviolet absorbers have so far been investigated JP-T-2002-524452 discloses a triazole ultraviolet absorber. Triazole ultraviolet absorbers having a maximum absorption wavelength in a long-wavelength ultraviolet light region have poor light resistance and their ultraviolet light shielding effect diminishes with time.

EP-A-1 213 283 teaches a biphenyl-substituted triazine compound of the following formula for inclusion in an organic polymer as a stabiliser to light, oxygen and heat:

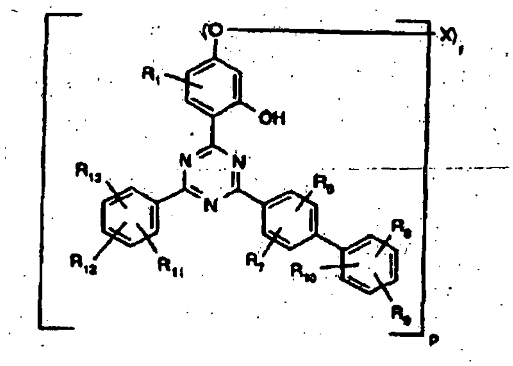

Materials used for solar cells which have been developed vigorously in recent years should be exposed to sunlight for long hours out of doors. Exposure of the materials to ultraviolet radiation for long hours however inevitably deteriorates their properties. There is therefore a demand for the development of compounds usable as an ultraviolet absorber effective for shielding a light even in a UV-A region and superior in light resistance to conventional ones.

SUMMARY OF THE INVENTION

An object of the invention is to provide a novel triazine compound effective for shielding an ultraviolet light even in a long wavelength region, having excellent light resistance, and useful as an ultraviolet absorber. Another object of the invention is to provide an ultraviolet absorber and a resin composition capable of not only improving ultraviolet light durability of, for example, a high molecular material but also capable of using the high molecular material as an ultraviolet light filter, thereby suppressing decomposition of another unstable compound; and capable of maintaining their long-wavelength ultraviolet light shielding effect for long hours.

The present inventors have investigated triazine compounds in detail and have found a compound effective for shielding a light of even a UV-A region, having unprecedentedly high light resistance, and having a conventionally unknown structure.

The above-described objects can be achieved by the following methods:

[1] A compound represented by the following formula (1):

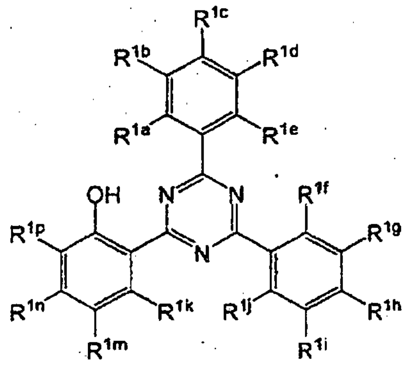

wherein $R^{1a}$, $R^{1b}$, $R^{1c}$, $R^{1d}$, and $R^{1e}$ each independently represents a hydrogen atom or a monovalent substituent other than OH with a proviso that at least one substituent has a positive $\sigma p$ value in Hammett equation and substituents may be coupled to each other to form a ring; $R^{1f}$, $R^{1g}$, $R^{1h}$, $R^{1i}$, and $R^{1j}$ each independently represents a hydrogen atom or a monovalent substituent other than OH with a proviso that substituents may be coupled to each other to form a ring; and $R^{1k}$, $R^{1m}$, $R^{1n}$, and $R^{1p}$ each independently represents a hydrogen atom or a monovalent substituent with a proviso that substituents may be coupled to each other to form a ring, and wherein both $R^{1c}$ and $R^{1h}$ are substituents having a positive $\sigma p$ value in the Hammet equation.

[2] The compound according to item [1], wherein

the monovalent substituent is a halogen atom, a substituted or unsubstituted alkyl group having 1 to 20 carbon atoms, a cyano group, a carboxyl group, a substituted or unsubstituted alkoxycarbonyl group, a substituted or unsubstituted carbamoyl group, a substituted or unsubstituted alkylcarbonyl group, a nitro group, a substituted or unsubstituted amino group, a hydroxyl group, an alkoxy group having 1 to 20 carbon atoms, a substituted or unsubstituted aryloxy group, a substituted or unsubstituted sulfamoyl group, a thiocyanate group, or a substituted or unsubstituted alkylsulfonyl group, and

when the monovalent substituent is further substituted by a substituent, the substituted is a halogen atom, an alkyl group having 1 to 20 carbon atoms, a cyano group, a carboxyl group, an alkoxycarbonyl group, a carbamoyl group, an alkylcarbonyl group, a nitro group, an amino group, a hydroxyl group, an alkoxy group having 1 to 20 carbon atoms, an aryloxy group, a sulfamoyl group, a thiocyanate group, or an alkylsulfonyl group.

The compound according to any one of items [1] to [2], wherein the $\sigma p$ value in Hammett equation falls within a range of from 0.1 to 1.2.

[4] The compound according to any one of items [1] to [3], wherein the substituent having a positive $\sigma p$ value in Hammett equation is a group selected from $COOR^r$, $CONR^s{}_2$, CN, $CF_3$, halogen atoms, $NO_2$, $SO_2R^t$, $SO_3M$, wherein $R^r$, $R^s$, and $R^t$ each independently represents a hydrogen atom or a monovalent substituent and M represents a hydrogen atom or an alkali metal.

[5] The compound according to any one of items [1] to [4], wherein the substituent having a positive $\sigma p$ value in Hammett equation is $COOR^r$, wherein $R^r$ represents a hydrogen atom or a monovalent substituent.

[6] The compound according to any one of items [1] to [4], wherein $R^{1c}$ represents CN.

[7] The compound according to any one of items [1] to [6], wherein $R^{1n}$ represents $OR^U$, wherein $R^U$ represents a hydrogen atom or a monovalent substituent.

[8] The compound according to item [7], wherein $R^U$ represents an alkyl group having 1 to 20 carbon atoms.

[9] The compound according to any one of items [1] to [8], which has pKa within a range of from -5.0 to -7.0.

[10] An ultraviolet absorber comprising a compound as described in any one of items [1] to [9].

[11] A resin composition comprising a compound as described in any one of items [1] to [9].

DETAILED DESCRIPTION OF THE INVENTION

A compound according to the invention can be used as an ultraviolet absorber. It exhibits high light fastness even in a long-wavelength ultraviolet light region so that by incorporating the compound in a resin composition for forming high molecular molded or formed products such as plastics and fibers, the resulting resin composition can have enhanced light stability.

(Compound represented by the formula (1))

An embodiment of the invention relates to a compound represented by the following formula (1):

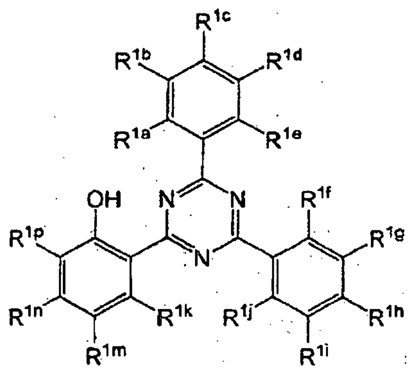

In the formula (1), $R^{1a}$, $R^{1b}$, $R^{1c}$, $R^{1d}$, and $R^{1e}$ each independently represents a hydrogen atom or a monovalent substituent other than OH with a proviso that at least one substituent has a positive σp value in Hammett equation and substituents may be coupled to each other to form a ring; $R^{1f}$, $R^{1g}$, $R^{1h}$, $R^{1i}$, and $R^{1j}$ each independently represents a hydrogen atom or a monovalent substituent other than OH with a proviso that substituents may be coupled to each other to form a ring; and $R^{1k}$, $R^{1m}$, $R^{1n}$, and $R^{1p}$ each independently represents a hydrogen atom or a monovalent substituent with a proviso that substituents may be coupled to each other to form a ring, Both $R^{1c}$ and $R^{1h}$ have a positive σp value in the Hammet equation.

$R^{1a}$, $R^{1b}$, $R^{1c}$, $R^{1d}$, and $R^{1e}$ each independently represents a hydrogen atom or a monovalent substituent other than OH with a proviso that at least $R^{1c}$ has a positive σp value in the Hammett equation.

It is preferred that one to three of the substituents represented by $R^{1a}$, $R^{1b}$, $R^{1c}$, $R^{1d}$, and $R^{1e}$ have a positive σp value in the Hammett equation and it is more preferred that one or two of the substituents have a positive σp value in the Hammett equation.

It is still more preferred that $R^{1c}$ represents a substituent having a positive σp value in the Hammett equation and $R^{1a}$, $R^{1b}$, $R^{1d}$, and $R^{1e}$ each represents a hydrogen atom.

$R^{1c}$ representing a substituent having a positive σp value in the Hammett equation is preferred because LUMO is stabilized with an electron withdrawing group, leading to shortening of an excited lifetime and improvement in light resistance.

Examples of the monovalent substituent (which will hereinafter be called "A") in the formula (1) include halogen atoms (such as fluorine, chlorine, bromine, and iodine), alkyl groups having 1 to 20 carbon atoms (such as methyl and ethyl), aryl groups having 6 to 20 carbon atoms (such as phenyl and naphthyl), a cyano group, a carboxyl group, alkoxycarbonyl groups (such as methoxycarbonyl), aryloxycarbonyl groups (such as phenoxycarbonyl), substituted or unsubstituted carbamoyl groups (such as carbamoyl, N-phenylcarbamoyl, and N,N-dimethylcarbamoyl), alkylcarbonyl groups (such as acetyl), arylcarbonyl groups (such as benzoyl), a nitro group, substituted or unsubstituted amino groups (such as amino, dimethylamino, anilino, and substituted sulfoamino), acylamino groups (such as acetamide and ethoxycarbonylamino), sulfonamide groups (such as methanesulfonamide), imide groups (such as succinimide and phthalimide), imino groups (such as benzylideneamino), a hydroxyl group, alkoxy groups having 1 to 20 carbon atoms (such as methoxy), aryloxy groups (such as phenoxy), acyloxy groups (such as acetoxy), alkylsulfonyloxy groups (such as methanesulfonyloxy), acrylsulfonyloxy groups (such as benzenesulfonyloxy), a sulfo group, substituted or unsubstituted sulfamoyl groups (such as sulfamoyl and N-phenylsulfamoyl), alkylthio groups (such as methylthio), arylthio groups (such as phenylthio), a thiocyanate group, alkylsulfonyl groups (such as methanesulfonyl), arylsulfonyl groups (such as benzenesulfonyl), and heterocyclic groups having 6 to 20 carbon atoms (such as pyridyl and morpholino).

The above-described substituent may be substituted further. If there are two or more substituents, they may be the same or different. Examples of the substituent include the monovalent substituents A exemplified above. The substituents may be coupled to each other to form a ring.

Examples of the ring formed by coupling of the substituents include a benzene ring, a pyridine ring, a pyrazine ring, a pyrimidine ring, a triazine ring, a pyridazine ring, a pyrrole ring, a pyrazole ring, an imidazole ring, a triazole ring, an oxazole ring, an oxadiazole ring, a thiazole ring, a thiadiazole ring, a furan ring, a thiophene ring, a selenophene ring, a silole ring, a germole ring, and phosphole ring.

As the monovalent substituent in the formula (1), halogen atoms, substituted or unsubstituted alkyl groups having 1 to 20 carbon atoms, a cyano group, a carboxyl group, substituted or unsubstituted alkoxycarbonyl groups, substituted or unsubstituted carbamoyl groups, substituted or unsubstituted alkylcarbonyl groups, a nitro group, substituted or unsubstituted amino groups, a hydroxyl group, substituted or unsubstituted alkoxy groups having 1 to 20 carbon atoms, substituted or unsubstituted aryloxy groups, substituted or unsubstituted sulfamoyl groups, a thiocyanate group, and substituted or unsubstituted alkylsulfonyl groups are preferred, of which $OR^U$ ($R^U$ representing a hydrogen atom or a monovalent substituent), alkyl groups and amide groups are more preferred, with $OR^U$ and alkyl groups being still

more preferred.

$R^U$ represents a hydrogen atom or a monovalent substituent and examples of the monovalent substituent include those belonging to Substituent A. Of these, linear or branched alkyl groups having 1 to 20 carbon atoms are preferred, with linear or branched alkyl groups having 1 to 6 carbon atoms being more preferred. Examples of the linear or branched alkyl groups having 1 to 6 carbon atoms include methyl, ethyl, n-propyl, i-propyl, n-butyl, i-butyl, s-butyl, t-butyl, n-pentyl, i-pentyl, t-pentyl, n-hexyl, i-hexyl, t-hexyl, n-octyl, t-octyl, and i-octyl. Of these, methyl and ethyl are preferred, with methyl being particularly preferred.

In the compound represented by the formula (1), $R^{tn}$ represents preferably a monovalent substituent, more preferably $OR^U$. It is still more preferred that $R^{1n}$ represents $OR^U$ and $R^{1k}$, $R^{1m}$, and $R^{1p}$ each represents a hydrogen atom, because a molar absorption coefficient increases, leading to enhancement of a shielding effect.

The substituent having a positive $\sigma p$ value in the Hammett equation in the formula (1) is preferably an electron withdrawing group having a $\sigma_p$ value of from 0.1 to 1.2. Specific examples of the electron withdrawing group having a $\sigma p$ value of 0.1 or greater include $COOR^r$ ($R^r$ representing a hydrogen atom or a monovalent substituent), $CONR^s{}_2$ ($R^s$ representing a hydrogen atom or a monovalent substituent), CN, halogen atoms, $NO_2$, $SO_2R^t$ ($R^t$ representing a hydrogen atom or a monovalent substituent), $SO_3M$ (M representing a hydrogen atom or an alkali metal), acyl groups, a formyl group, acyloxy groups, acylthio groups, alkyloxycarbonyl groups, aryloxycarbonyl groups, dialkylphosphono groups, diarylphosphono groups, dialkylphosphinyl groups, diarylphosphinyl groups, phosphoryl groups, alkylsulfinyl groups, arylsulfinyl groups, acylthio groups, sulfamoyl groups, a thiocyanate group, a thiocarbonyl group, an imino group, an N-atom substituted imino group, carboxy groups (or salts thereof), alkyl groups substituted with at least two halogen atoms (such as $CF_3$), alkoxy groups substituted with at least two halogen atoms, aryloxy groups substituted with at least two halogen atoms, acylamino groups, alkylamino groups substituted with at least two halogen atoms, alkylthio groups substituted with at least two halogen atoms, aryl groups substituted with another electron withdrawing group having a $\sigma p$ value of 0.2 or greater, heterocyclic groups, halogen atoms, an azo group, and a selenocyanate group. The $\sigma p$ value in the Hammett equation is described specifically in Hansch, C., Leo, A., and Taft, R.W:, Chem. Rev. 91, 165-195(1991).

The substituent having a positive $\sigma p$ value in the Hammett equation in the formula (1) is more preferably $COOR^r$, $CONR^s{}_2$, CN, $CF_3$, a halogen atom, $NO_2$, $SO_2R_t$, or $SO_3M$ ($R^r$ and $R^s$ each independently represents a hydrogen atom or a monovalent substituent, and M represents a hydrogen atom or an alkali metal). Of these, $COOR^r$ or CN is more preferred, with $COOR^r$ being still more preferred because it provides excellent light resistance and solubility.

$R^r$ represents a hydrogen atom or a monovalent substituent and examples of the monovalent substituent include those belonging to Substituent A. Of these, linear or branched alkyl groups having 1 to 20 carbon atoms are preferred, with linear or branched alkyl groups having 1 to 6 carbon atoms being more preferred. Examples of the linear or branched alkyl groups having 1 to 6 carbon atoms include methyl, ethyl, n-propyl, i-propyl, n-butyl, i-butyl, s-butyl, t-butyl, n-pentyl, i-pentyl, t-pentyl, n-hexyl, i-hexyl, t-hexyl, t-octyl, i-octyl, and n-octyl. Of these, methyl and ethyl are preferred, with methyl being particularly preferred.

In the compound represented by the formula (1), $R^{1c}$ is preferably any one of $COOR^r$, $CONR^s{}_2$, CN, $CF_3$, a halogen atom, $NO_2$, $SO_2R^t$, or $SO_3M$, more preferably $COOR^r$ or CN. From the viewpoint of light resistance, $R^{1c}$ is preferably CN.

$R^{1h}$ represents a substituent having a positive $\sigma p$ value in the Hammett equation, such

that both of $R^{1c}$ and $R^{1h}$ represent substituents having a positive $\sigma p$ value in the Hammett equation, because such a compound of the formula (1) has excellent light resistance.

The compound represented by the formula (1) has preferably pKa of from -5.0 to -7.0, more preferably -5.2 to -6.5, particularly preferably -5.4 to -6.0.

Specific examples of the compound represented by the formula (1) will next be described but the invention is not limited thereto.

In the following specific examples, Me represents a methyl group and $C_6H_{13}$ represents an n-hexyl group.

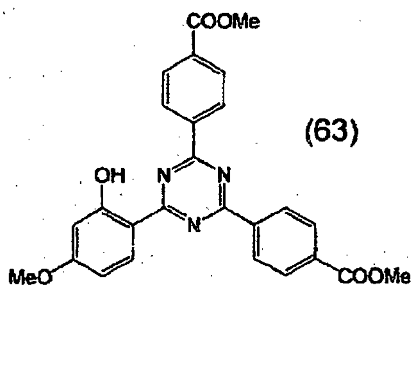
(63)

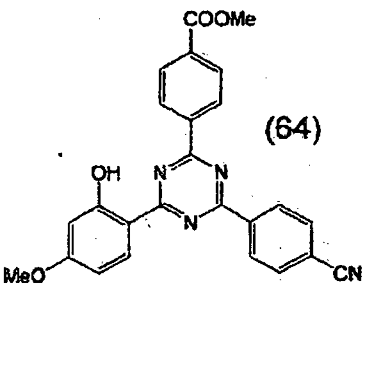
(64)

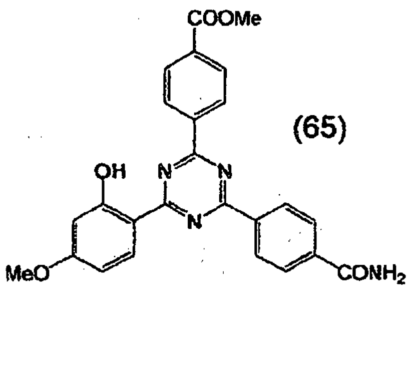
(65)

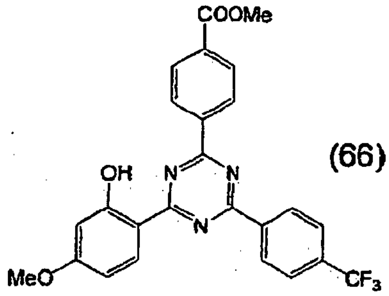

(66)

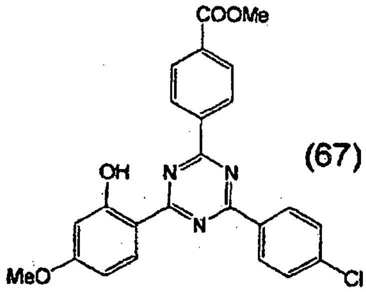

(67)

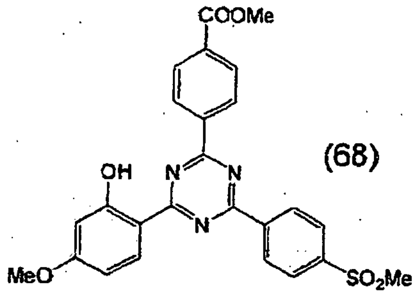

(68)

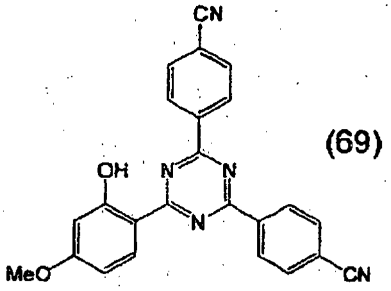

(69)

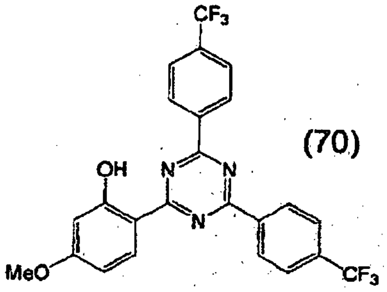

(70)

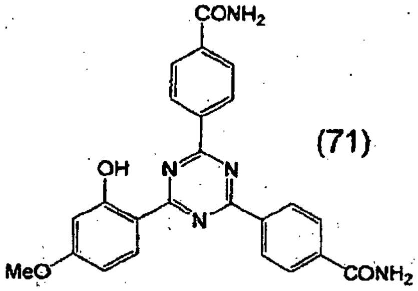

(71)

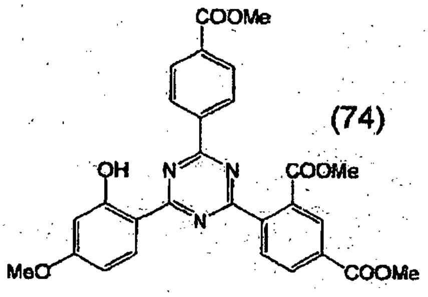

(74)

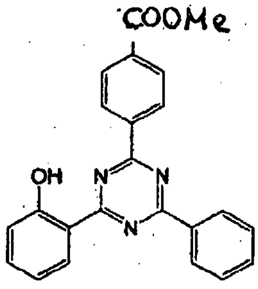

(75)

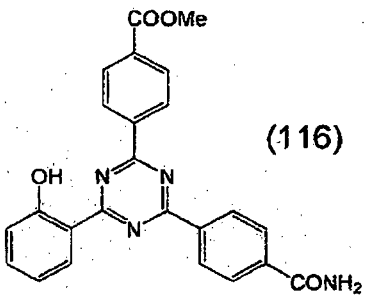

(116)

The compound represented by the formula (1) may become a tautomer, depending on its structure and environment where the compound is placed. In an exemplary embodiment of the invention, the tautomer is described as

being one of typical tautomers, but tautomers different from that described herein are also encompassed by the invention.

The compound represented by the formula (1) may contain an isotope (such as $^2H$, $^3H$, $^{13}C$, $^{15}N$, $^{17}O$, or $^{18}O$).

The compound represented by the formula (1) can be synthesized using any process.

It can be synthesized referring to, for example, publicly known patent documents or non-patent documents, for example, JP-A-7-188190, JP-A-11- 315072, JP-A- 2001-220385, or Dyestuffs & chemicals, 40(12), 325-339 (1995). More specifically, Exemplified compound (19) can be synthesized by reacting 4-methoxysalicylamide, 3,5-bis(trifluoromethyl)benzoyl chloride, and benzamidine hydrochloride.

The compound of the invention is particularly suited for stabilizing organic materials, thereby making them resistant to damages due to light, oxygen or heat. Of these, the compound of the invention represented by the formula (1) can be used suitably as a light stabilizer, particularly, an ultraviolet absorber.

(Ultraviolet absorber)

An ultraviolet absorber composed of the compound represented by the formula (1) will next be described.

The ultraviolet absorber of the invention includes a compound represented by the formula (1). The compound of the invention represented by the formula (1) has, at a specific position thereof, a substituent having a positive $\sigma p$ value in the Hammett equation so that LUMO is stabilized by an electron withdrawing group. As a result, the compound has a short excited lifetime and excellent light resistance. When a known triazine compound is used as an ultraviolet absorber, using it for long hours causes decomposition and yellowing, thus having an adverse effect.

The compound of the invention represented by the formula (1), on the other hand, does not cause decomposition and therefore does not cause yellowing even when used for long hours, because it has excellent light resistance.

The compounds represented by the formula (1) may be used either singly or in combination.

The ultraviolet absorber of the invention may be used in any form. Examples of the using form include a liquid dispersion, a solution, and a resin composition.

Although the maximum absorption wavelength of the ultraviolet absorber of the invention is not particularly limited, it is preferably from 250 to 400 nm, more preferably from 280 to 380 nm. The half band width is preferably from 20 to 100 nm, more preferably from 40 to 80 nm.

The maximum absorption wavelength and half band width as specified by the invention can be readily measured by those skilled in the art. The measuring method and the like are described, for example, in The fourth series of Experimental Chemistry 7: Spectroscopy II, pp. 180 to 186 (published by Maruzen in 1992). Described specifically, a sample is dissolved in a suited solvent and the maximum absorption wavelength and the half band width of the resulting solution are measured with a spectrophotometer by using a sample cell and a control cell, each made of quartz or glass. The solvent used for the measurement is required to have, in addition to the adequate solubility for the sample, no absorption in a measurement wavelength region, have a small interaction with a solute molecule, and have not remarkable volatility. Any solvent can be selected for use insofar as it can satisfy the above-described conditions. In the invention, measurement is performed by using ethyl acetate (EtOAc) as the solvent.

As the maximum absorption wavelength and half band width of the compound in the invention, those obtained by preparing a solution having a concentration of about $5 \times 10^{-5}$ mol/dm$^{-3}$ and measuring with a quartz cell having an optical path length of 10 mm are used.

With respect to the spectrum half band width, a description can be found, for example, in The fourth series of Experimental Chemistry 3: Basic Operation III, p. 154 (published by Maruzen in 1991). In the book, the half band width is described using an example where a frequency scale is plotted along the abscissa, while the half band width in the invention is described in an example where a wavelength scale is plotted along the abscissa and the half band width is expressed in unit of nm. More specifically, the half band width means the width of an absorption band half of the absorbance in the maximum absorption wavelength and it is used as a value expressing the shape of an absorption spectrum. A spectrum having a small half band width is a sharp spectrum, while a spectrum having a large half band width is a broad spectrum. Ultraviolet absorbing compounds providing a broad spectrum have absorption also in a wide region on the long wavelength side from the maximum absorption wavelength so that in order to effectively shield a light in a long-wavelength ultraviolet light region without causing yellow-tinge coloring, ultraviolet absorbing compounds having a spectrum with a small half band width are preferred.

As described in Sumio Tokita, Chemistry Seminar 9: Color Chemistry, pp. 154-155 (published by Maruzen in 1982), the intensity of light absorption, that is, the intensity of an oscillator is proportional to the integration of a molar absorption coefficient. When the absorption spectrum shows good symmetry, the intensity of an oscillator is proportional to the product of an absorbance and a half band width at the maximum absorption wavelength (with the proviso that the half band width in this case is a value when a wavelength scale is plotted along the abscissa). This means that when two compounds have the same transition moment, the compound having a spectrum with a smaller half band width has a larger absorbance at the maximum absorption wavelength. Such an ultraviolet absorbing compound can effectively shield a light in a region around the maximum absorption wavelength even if the using amount of it is small. It however

cannot shield a light in a wide region because a drastic reduction in absorbance occurs at a wavelength a little distant from the maximum absorption wavelength.

The ultraviolet absorber has preferably a molar absorption coefficient, at the maximum absorption wavelength, of 20000 or greater, more preferably 30000 or greater, especially preferably 50000 or greater. When the molar absorption coefficient is 20000 or greater, a sufficient absorption efficiency per mass of the ultraviolet absorber can be attained so that a using amount of it for completely absorbing light in the ultraviolet region can be reduced. It is preferred from the standpoint of preventing skin irritation and accumulation in a living body and causing less bleed-out. It is to be noted that for the definition of the molar absorption coefficient, that described in New Series of Experimental Chemistry 9: Analytical Chemistry [II], p. 244, ed. by Nippon Chemistry Association, (published by Maruzen in 1977) is used and it can be determined together with the above-described maximum absorption wavelength and half band width value when they are determined.

The ultraviolet absorber of the invention (which may also be called "the ultraviolet absorber", simply) can also be used in the form of a dispersion obtained by dispersing the ultraviolet absorber in a dispersion medium. An ultraviolet absorber dispersion containing the ultraviolet absorber of the invention will next be described.

The medium in which the ultraviolet absorber of the invention is to be dispersed is not limited. Examples include water, an organic solvent, a resin, and a solution of a resin. They may be used either singly or in combination.

Examples of the organic solvent serving as the dispersion medium used in the invention include hydrocarbon solvents such as pentane, hexane, and octane, aromatic solvents such as benzene, toluene, and xylene, ether solvents such as diethyl ether and methyl-t-butyl ether, alcohol solvents such as methanol, ethanol, and isopropanol, ester solvents such as acetone, ethyl acetate and butyl acetate, ketone solvents such as methyl ethyl ketone, nitrile solvents such as acetonitrile and propionitrile, amide solvents such as N,N-dimethylformamide, N,N-dimethylacetamide, and N-methylpyrrolidone, sulfoxide solvents such as dimethyl sulfoxide, amine solvents such as triethylamine and tributylamine, carboxylic acid solvents such as acetic acid and propionic acid, halogen solvents such as methylene chloride and chloroform, and heterocyclic solvents such as tetrahydrofuran and pyridine. These solvents may be used in combination at any ratio.

Examples of the resin serving as the dispersion medium to be used in the invention include thermoplastic resins and thermosetting resins conventionally used for the production of various publicly known molded or formed products, sheets, films, and the like. Examples of the thermoplastic resins include polyethylene resins, polypropylene resins, poly(meth)acrylate resins, polystyrene resins, styrene-acrylonitrile resins, acrylonitrile-butadiene-styrene resins, polyvinyl chloride resins, polyvinylidene chloride resins, polyvinyl acetate resins, polyvinyl butyral resins, ethylene-vinyl acetate copolymers, ethylene-vinyl alcohol resins, polyethylene terephthalate resins (PET), polybutylene terephthalate resins (PBT), liquid-crystal polyester resins (LCP), polyacetal resins (POM), polyamide resins (PA), polycarbonate resins, polyurethane resins, and polyphenylene sulfide resins (PPS). They may be used either singly or as a polymer blend or polymer alloy of two or more thereof. These resins may be used as a thermoplastic molding or forming material obtained by incorporating, in a natural resin, a filler such as glass fibers, carbon fibers, semi-carbonized fibers, cellulose fibers, or glass beads, or a flame retardant. If necessary, conventionally used additives for resins such as fine powders of a polyolefin resin, a polyolefin wax, an ethylenebisamide wax, and a metal soap may be use either singly or in combination.

Examples of the thermosetting resins include epoxy resins, melamine resins, and unsaturated polyester resins. They may also be used as a thermosetting molding or forming material obtained by incorporating, in addition to a natural resin, a filler such as glass fibers, carbon fibers, semi-carbonized fibers, cellulose fibers, or glass beads, or a flame retardant.

The dispersion containing the ultraviolet absorber may further contain a dispersant, an antifoaming agent, a preservative, an anti-freezing agent, or a surfactant. It may further contain any other compound. Examples include dyes, pigments, infrared absorbers, perfumes, polymerizable compounds, polymers, inorganic materials, and metals.

As an apparatus for obtaining a dispersion containing the ultraviolet absorber of the invention, a high-speed stirring type disperser having a high shear force or a disperser providing a high-strength ultrasonic energy can be used. Specific examples include a colloid mill, a homogenizer, a capillary emulsifying machine, a liquid siren, an electromagnetic distortion system ultrasonic wave generator, and an emulsifying apparatus with a Pohlmann whistle. In the high-speed stirring type disperser preferably used in the invention, a main portion executing a dispersing action rotates at a high speed (from 500 to 15,000 rpm, preferably 2,000 to 4,000 rpm) in a liquid. The high-speed stirring type dispersing machine to be used in the invention is also called "dissolver" or "high-speed impeller disperser". As described in JP-A-55-129136, one of the preferred examples of it has a high-speed turning shaft equipped with an impeller obtained by alternately folding a saw-teeth shaped plate in a perpendicular direction.

Upon preparing an emulsified dispersion containing a hydrophobic compound, various processes can be employed. For example, in order to dissolve a hydrophobic compound in an organic solvent, the hydrophobic compound is dissolved in a solvent or a mixture of two or more selected freely from high-boiling point organic materials, water-immiscible low boiling point organic solvents and water-miscible organic solvents and then the resulting solution is dispersed in water or an aqueous solution of a hydrophilic colloid in the presence of a surface active compound. Mixing

of a water insoluble phase containing the hydrophobic compound and a water phase may be performed by either a so-called forward mixing method of adding the water insoluble phase to the water phase under stirring or a mixing method of adding these phases reversely.

The ultraviolet absorber of the invention may be used in the form of a solution obtained by dissolving in a liquid medium. A solution containing the ultraviolet absorber of the invention will next be described.

A liquid in which the ultraviolet absorber of the invention is to be dissolved is not limited. Examples include water, an organic solvent, a resin, a solution of a resin, and the like. Examples of the organic solvent, the resin, or the solution of a resin are similar to those described as the above-described dispersion medium. They may be used either singly or in combination.

The solution of the ultraviolet absorber of the invention may contain, in addition, any compound. Examples include dyes, pigments, infrared absorbers, perfumes, polymerizable compounds, polymers, inorganic materials, and metals. Compounds other the ultraviolet absorber of the invention are not necessarily be dissolved.

Although the content of the ultraviolet absorber of the invention in the ultraviolet-absorber-containing solution cannot be determined in a wholesale manner because it differs, depending on the using purpose and usage, it may be any, depending on the using purpose. The content is preferably from 0.001 to 30 mass% (weight%), more preferably from 0.01 to 10 mass%, based on the total weight of the solution. It is also possible to prepare a high-concentration solution and then dilute it as desired. A diluting solvent may be selected freely from the above-described solvents.

Examples of materials stabilized by the ultraviolet absorber of the invention include dyes, pigments, foods, beverages, health care products, vitamin preparations, pharmaceuticals, inks, oils, fats, waxes, surface coating, cosmetics, photographic materials, fabrics and dyes therefor, plastic materials, rubbers, paints, resin compositions, and high-molecular additives.

When the ultraviolet absorber of the invention is used, it may be used in any mode. The ultraviolet absorber of the invention may be used either singly or as a composition. Using it as a composition is more preferred. The composition is still more preferably a resin composition containing the ultraviolet absorber of the invention. The resin composition containing the ultraviolet absorber of the invention will next be described.

(Resin composition)

A resin composition containing an ultraviolet absorber of the invention contains a resin. The resin composition containing the ultraviolet absorber of the invention may be formed by dissolving a resin in an arbitrary solvent.

The ultraviolet absorber of the invention can be incorporated in a resin composition in various manners. When the ultraviolet absorber of the invention has compatibility with the resin composition, the ultraviolet absorber of the present invention can be added directly to the resin composition. After dissolving the ultraviolet absorber of the invention in an auxiliary solvent having compatibility with the resin composition, the resulting solution may be added to the resin composition. Alternatively, the ultraviolet absorber of the invention may be dispersed in a high-boiling point organic solvent or polymer and the resulting dispersion may be added to the resin composition.

(High-boiling point organic solvent)

The high-boiling point organic solvent has a boiling point of preferably 180°C or greater, more preferably 200°C or greater. The melting point of the high-boiling point organic solvent is preferably 150°C or less, more preferably 100°C or less. Examples of the high-boiling point organic solvent include phosphoric acid esters, phosphonic acid esters, benzoic acid esters, phthalic acid esters, fatty acid esters, carbonic acid esters, amides, ethers, halogenated hydrocarbons, alcohols and paraffin. Of these, phosphoric acid esters, phosphonic acid esters, phthalic acid esters, benzoic acid esters, and fatty acid esters are preferred.

The ultraviolet absorber of the invention can be added with reference to JP-A-58-209735, JP-A-63-264748, JP-A-4-191851, and JP-A-8-272058, and British Patent No. 2016017A.

(Resin)

The resin used for the resin composition will next be described. The resin may be either a natural polymer or a synthetic polymer. Examples include polyolefins (such as polyethylene, polypropylene, polyisobutylene, poly(1-butene), poly-4-methylpentene, polyvinylcyclohexane, polystyrene, poly(p-methylstyrene), poly($\alpha$-methylstyrene), polyisoprene, polybutadiene, polycyclopentene, and polynorbomene), copolymers of a vinyl monomer (e.g., ethylene/propylene copolymer, ethylene/methylpentene copolymer, ethylene/heptene copolymer, ethylene/vinylcyclohexane copolymer, ethylene/cycloolefin copolymer (ex. ethylene/cycloolefin copolymer (COC) such as ethylene/norbornene copolymer), propylene/butadiene copolymer, isobutylene/isoprene copolymer, ethylene/vinylcyclohexene copolymer, ethylene/alkyl acrylate copolymer, and ethylene/alkyl methacrylate copolymer), acrylic polymers (such as polymethacrylate, polyacrylate,

polyacrylamide, and polyacrylonitrile), polyvinyl chloride, polyvinylidene chloride, polyvinyl fluoride, polyvinylidene fluoride, vinyl chloride/vinyl acetate copolymer, polyethers (such as polyalkylene glycol, polyethylene oxide, and polypropylene oxide), polyacetals (such as polyoxyethylene), polyamide, polyimide, polyurethane, polyurea, polyesters (such as polyethylene terephthalate and polyethylene naphthalate), polycarbonate, polyketone, polysulfone polyether ketone, phenol resin, melamine resin, cellulose esters (such as diacetyl cellulose, triacetyl cellulose (TAC), propionyl cellulose, butyryl cellulose, acetyl propionyl cellulose, and nitrocellulose), polysiloxane, and natural polymers (such as cellulose, rubber, and gelatin).

The resin to be used in the invention is preferably a synthetic polymer, more preferably polyolefin, acrylic polymer, polyester, polycarbonate, or cellulose ester. Of these, polyethylene, polypropylene, poly(4-methylpentene), polymethyl methacrylate, polycarbonate, polyethylene terephthalate, polyethylene naphthalate, polybutylene terephthalate, and triacetyl cellulose are particularly preferred.

The resin to be used in the invention is preferably a thermoplastic resin.

The ultraviolet absorber of the invention can be incorporated in a resin composition in an arbitrary amount necessary for providing it with a desired performance. The content differs depending on the compound or resin to be used for preparing the resin composition, but can be determined as needed. The content in the resin composition is preferably more than 0 mass% and 20 mass% or less, more preferably more than 0 mass% and 10 mass% or less, still more preferably 0.05 mass% or more and 5 mass% or less. The contents within the above-described range are preferred because they can give a sufficient ultraviolet light shielding effect and can suppress bleed-out.

The resin composition of the present invention may contain, in addition to the above-described high molecular substance and ultraviolet absorber, an arbitrary additive such as antioxidant, light stabilizer, processing stabilizer, anti-aging agent, and compatibilizing agent as needed.

The resin composition containing the ultraviolet absorber of the invention is applicable to any application where synthetic resin is used. It is particularly preferably applicable to applications which may be exposed to light such as sunlight or ultraviolet light. Specific examples thereof include glass alternatives and their surface-coating materials; coating materials for the window glass, lighting glass and light-protecting glass of house, facility, and transport apparatus; window films of house, facility, and transport apparatus; interior and exterior materials of house, facility, and transport apparatus, paints for the interior and exterior materials, and films formed by the paints; alkyd resin lacquer paints and paint films formed by the paints; acryl lacquer paints and paint films formed by the paints; materials for ultraviolet-emission sources such as fluorescent lamp and mercury lamp; materials for precision machines and electric and electronic devices; materials for shielding electromagnetic and other waves emitted from various displays; containers or packaging materials for foods, chemicals and drugs; special packages such as bottle, box, blister, and cup; discoloration inhibitors for compact disk coating, agricultural and industrial sheet or film, print, colored products, dyes and pigments; protective film for polymer supports (such as plastic parts such as mechanical and automotive parts); print over-coating, inkjet medium film, delustered laminate film, optical light film, safety glass/front glass intermediate layer, electrochromic/photochromic film, over-lamination film, solar-heat-controlling film, cosmetics such as anti-sunbum cream, shampoo, rinse, and hair styling agent; apparel fiber products such as sport wear, stockings and cap and the fibers; home interior products such as curtain, carpet and wall paper; medical devices such as plastic lens, lenses of spectacles, contact lens and artificial eye; optical materials such as optical filter, backlight display film, prism, mirror, and photographic material; mold film, transfer-type sticker, anti-graffiti film, stationery products such as tape and ink; and display plates and devices and the surface-coating agents thereof.

High-molecular molded or formed products available from the resin composition of the invention may be in any form such as flat film, powder, spherical particle, crushed particle, bulky continuous particle, fiber, tube, hollow yarn, granule, plate, or porous.

Since the high molecular material of the invention contains the ultraviolet absorber of the invention, it has excellent light resistance (ultraviolet light fastness) and causes neither precipitation of the ultraviolet absorber nor bleed-out due to long-term use. The resin composition of the invention has excellent long-wavelength ultraviolet light absorption performance so that it can be used as an ultraviolet absorbing filter or container and can protect compounds susceptible to ultraviolet light. For example, the high-molecular substance can be formed or molded into a molded or formed product (container, or the like) by an arbitrary method such as extrusion or injection molding. It is also possible to obtain a molded or formed product coated with an ultraviolet absorbing film including the resin composition of the invention by applying and drying a solution of the high molecular substance to a separately manufactured molded or formed product.

When the resin composition of the invention is used as an ultraviolet absorbing filter or ultraviolet absorbing film, the high molecular substance is preferably transparent. Examples of the transparent resin include cellulose esters (such as diacetyl cellulose, triacetyl cellulose (TAC), propionyl cellulose, butyryl cellulose, acetyl propionyl cellulose, and nitrocellulose), polyamides, polycarbonates, polyesters (such as polyethylene terephthalate, polyethylene naphthalate, polybutylene terephthalate, poly-1,4-cyclohexane dimethylene terephthalate, polyethylene-1,2-diphenoxyethane-4,4'-dicarboxylate, and polybutylene terephthalate), polystyrenes (such as syndiotactic polystyrene), polyolefins (such as polyethylene, polypropylene, and polymethylpentene), polymethyl methacrylate, syndiotactic polystyrene, polysulfones, polyether sulfones, polyether ketones, polyether imides, and polyoxyethylene. Of these, cellulose esters, polycarbonates, polyesters, polyolefins, and acrylic resins are preferred, with polycarbonates and polyester being more preferred. Polyesters are still more preferred and polyethylene terephthalate is particularly preferred. The high molecular molded or formed product obtained from the resin composition of the invention can also be used as a transparent support. The transparent support has a transmission of 80% or greater, more preferably 86% or greater.

In the invention, two or more compounds represented by the formula (1) but different in structure may be used in combination as the ultraviolet absorber. The compound represented by the formula (1) may be used in combination with one or more ultraviolet absorbers having a structure other than the structure of the compound (1). Combined use of two or more (preferably three) ultraviolet absorbers different in fundamental skeleton structure makes it possible to absorb ultraviolet light of a wide wavelength range. Combined use of two or more ultraviolet absorbers is effective for stabilizing the dispersion state of the ultraviolet absorbers. As the ultraviolet absorber having a structure other than that of the formula (1), any one is usable. Examples include triazine, benzotriazole, benzophenone, merocyanine, cyanine, dibenzoylmethane, cinnamic acid, cyanoacrylate, and benzoic acid ester compounds. More specific examples include ultraviolet absorbers described, for example, in Fine Chemical, May, 28-38(2004), Development of high-molecular functional additives, p. 96 to 140 (published by Toray Research Center in 1999), and Yasuichi Okatsu, ed., Development of high molecular additive and environmental measures, p. 54 to 64, (published by CMC in 2003).

The ultraviolet absorber having a structure other than that of the formula (1) is preferably a benzotriazole compound, a benzophenone compound, a salicylic acid compound, a benzoxazinone compound, a cyanoacrylate compound, a benzoxazole compound, a merocyanine compound, or a triazine compound, more preferably a benzoxazinone compound, a benzotriazole compound, a benzophenone compound, or a triazine dompound, particularly preferably a benzoxazinone compound. The ultraviolet absorbers having a structure other than that of the formula (1) is described specifically in paragraphs 0117 to 0121 of Japanese Patent Application No. 2008-273950 and the materials described therein can also be used in the invention.

As described above, in the invention, combined use of the compound represented by the formula (1) and a benzoxazinone compound is preferred. The compound represented by the formula (1) has excellent light resistance even in a long wavelength region so that it is effective for preventing deterioration of benzoxazinone capable of shielding light of even a longer wavelength region. Using it together with a benzoxazinone compound is preferred because it can keep, for long hours, the effect of shielding light of a longer wavelength region.

In the invention, single use of the ultraviolet absorber of the invention can provide an ultraviolet light shielding effect sufficient for practical use. When further strictness is required, a white pigment having a strong shielding power such as titanium oxide may be used in combination. When an appearance or color tone is important or if desired, a trace amount (0.05 mass% or less) of a colorant can be used in combination. A fluorescent brightener may be used in combination for applications in which transparency is important or which are required to be white. Examples of the fluorescent brightener include commercially available ones and compounds of the formula (1) and Specific Compound Examples 1 to 35 described in JP-A-2002-53824.

The present invention will hereinafter be described in further detail by Examples. It should however be borne in mind that the invention is not limited to them.

Example 9

(Synthesis of Exemplified compound (63))

To 10.0 g of phenyl 4-methoxysalicylate were added 100 mL of methanol, 15.8 g of a 28% sodium methoxide methanol solution, and 14.6 g of methyl 4-amidinobenzoate hydrochloride. The resulting solution was stirred at 60°C for 5 hours. After cooling to room temperature, 0.2 mL of 35% hydrochloric acid was added. The solid thus obtained was filtered and washed with water and methanol to yield 18.0 g of Exemplified compound (63) (yield: 93%).
MS: m/z 472 (M+).

Example 10

(Synthesis of Exemplified compound (64))

To 4.2 g of methyl 4-amidinobenzoate hydrochloride were added 100 mL of methanol and 3.8 g of a 28% sodium methoxide methanol solution. To the resulting solution was added 5.0 g of Synthetic intermediate D and the resulting mixture was stirred at 60°C for 3 hours. After the reaction mixture was cooled to room temperature, 0.2 mL of 35% hydrochloric acid was added. The solid thus obtained was filtered and washed with water and methanol to yield 7.5 g of Exemplified compound (64) (yield: 95%).
MS: m/z 439 (M+).

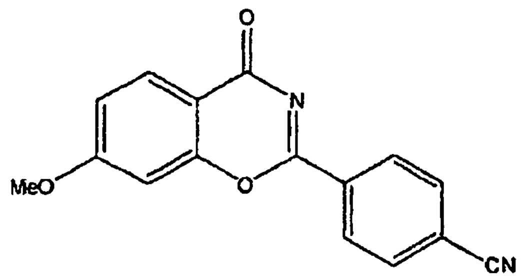

(Synthetic intermediate D)

Example 11

(Synthesis of Exemplified compound (65)).

To 3.6 g of 4-amidinobenzamide hydrochloride were added 100 mL of methanol and 3.4 g of a 28% sodium methoxide methanol solution. To the resulting solution was added 5.0 g of Synthetic intermediate B and the resulting mixture was stirred at 60°C for 3 hours. After the reaction mixture was cooled to room temperature, 0.2 mL of 35% hydrochloric acid acid was added. The solid thus obtained was filtered and washed with water and methanol to yield 6.9 g of Exemplified compound (65) (yield: 94%).
MS: m/z 457 (M+).

Reference Example 12

(Synthesis of Exemplified compound (75))

Acetonitrile (80 mL) and 44.4 g of DBU were added to 20.0 g of salicylamide to dissolve it To the resulting solution was added 29.0 g of methyl 4-(chloroformyl)benzoate and the resulting mixture was stirred at room temperature for 24 hours. To the reaction mixture were added 100 mL of water and 20 mL of hydrochloric acid. The solid thus obtained was filtered and washed with water to yield 40.0 g of Synthetic intermediate K (yield: 92%).

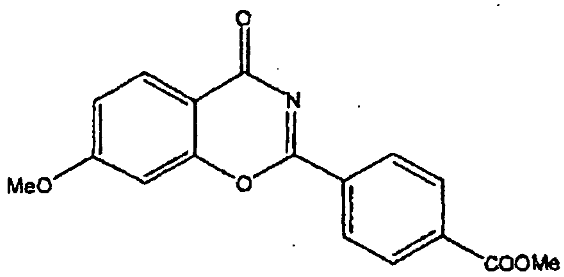

(Synthetic intermediate B)

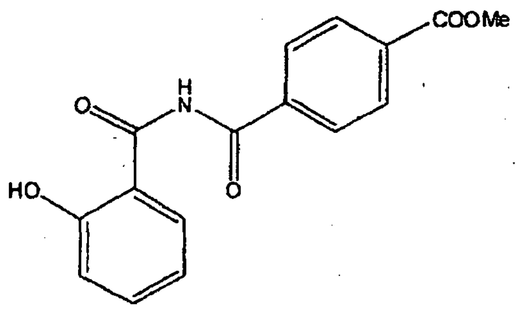

(Synthetic intermediate K)

To 20.0 g of Synthetic intermediate K were added 200 mL of acetonitrile and 9.4 g of sulfuric acid. The resulting mixture was stirred at 90°C for 4 hours. To the reaction mixture was added 80 mL of triethylamine, followed by cooling to room temperature. The solid thus obtained was filtered and washed with water to yield 18.2 g of Synthetic intermediate L (yield: 97%).

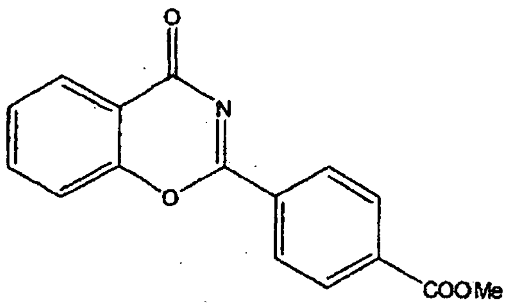

(Synthetic intermediate L)

To 3.1 g of benzamidine hydrochloride were added 100 mL of methanol and 3.8 g of a 28% sodium methoxide methanol solution. To the resulting solution was added 5.0 g of Synthetic intermediate L and the resulting mixture was stirred at 60°C for 3 hours. After the reaction mixture was cooled to room temperature, 0.2 mL of 35% hydrochloric acid was added. The solid thus obtained was filtered and washed with water and methanol to yield 6.4 g of Exemplified compound (75) (yield: 94%).
MS: m/z 384 (M+).

Example 26

(Synthesis of Exemplified compound (116))

In a manner similar to that employed for the Synthesis of Exemplified compound (75) described in Reference Example 12 except that benzamidine hydrochloride used as a raw material was replaced with 4-amidinobenzamide hydrochloride, Exemplified compound (116) was synthesized.
$^1$H NMR ($CDCl_3$): δ3.93(3H), δ7.07-7.13(2H), δ7.58-7.63(2H), δ8.13-8.15(2H), δ8.20-824(3H), δ8.63-8.73(5H), δ12.88(1H) λmax=346 nm (EtOAc).

<Measurement method of pKa>

Exemplified compound (63) was dissolved in acetonitrile to give its absorption coefficient of 1. To the resulting solution was added dropwise 70% perchloric acid (using acetic acid as a solvent) to gradually change the pH of the resulting solution. At that time, a solution absorption spectrum was measured and a ratio of a triazine-free compound and a proton adduct at each pH was computed from the absorbance at λmax. The value of pKa was determined from a point at which the ratio became 1. Similarly, the pKa of each of Exemplified compound (64), Exemplified compound (65), Exemplified compound (75), Exemplified compound (116), Comparative compound A, and Comparative compound B. The absorption spectrum was measured using a spectrophotometer "UV-3600" (trade name; product of Shimadzu Corp), while pH was measured using a pH meter "HM60G" (trade name; product of Toa Denpa Kogyo).

<Preparation and evaluation of polymer film>

A binder solution was prepared by dissolving 22 mass% of a PMMA resin ("DIANAL BR-80", trade name; product of Mitsubishi Rayon) in methylene chloride. A coating solution was then prepared by dissolving 0.2 mass% of Exemplified compound (63) in the resulting binder solution. Using glass as a base material, the resulting coating solution was applied thereto with a 200 μm blade and dried at 100°C for 10 minutes into a 50-μm thick coating to prepare a film. Similarly, a film was prepared using each of Exemplified compound (64), Exemplified Exemplified compound (116), Comparative compound A, and Comparative compound B. The absorbance of the film was measured with a spectrophotometer "UV-3600" (trade name; product of Shimadzu Corp). The film was exposed to light of a metal halide lamp ("Eye super UV tester", trade name; product of Iwasaki Electric) under the conditions of illuminance of 90 mW/cm$^2$, temperature of 63°C, and humidity of 50%. The remaining amount of each compound 400 hours after exposure was measured. The remaining amount was computed in accordance with the following equation:

Remaining amount (%)=100×(100-(transmittance after exposure))/(100-(transmittance before exposure))

The transmittance is a value measured at the maximum absorption wavelength of each compound. The results are shown in Table 1.

Table 1

| Sample No. | Compound | Remaining amount (%) | pKa | |
|---|---|---|---|---|
| 9 | Exemplified compound (63) | 99 | -5.5 | Invention |
| 10 | Exemplified compound (64) | 99 | -5.6 | Invention |
| 11 | Exemplified compound (65) | 99 | -5.4 | Invention |
| 12 | Exemplified compound (75) | 99 | -5.8 | Reference |
| 26 | Exemplified compound (116) | 99 | -5.6 | Invention |
| 31 | Comparative compound A | 63 | -4.9 | Comparative example |
| 32 | Comparative compound B | 21 | -4.4 | Comparative example |

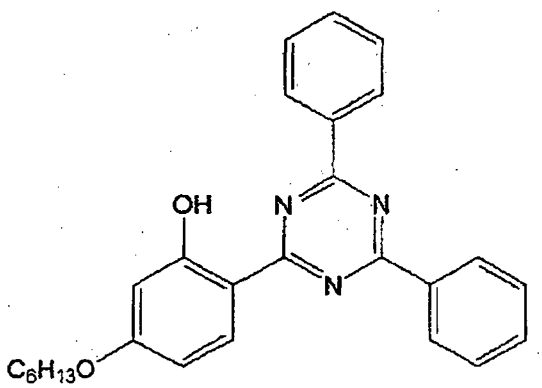

TINUVIN1577FF

(Comparative compound A)

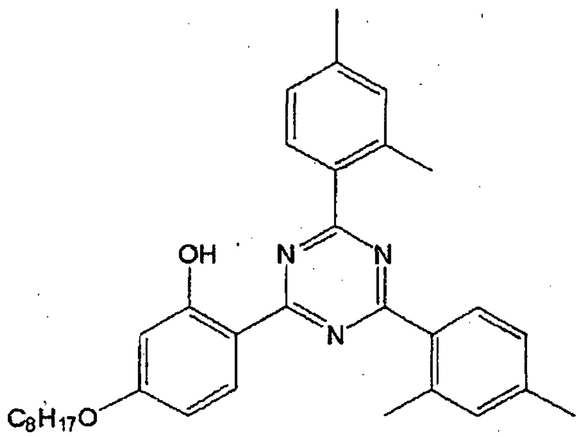

CYASORB UV-1164

(Comparative compound B)

It has been found from the results of Table 1 that compared with the comparative compounds (existing ultraviolet absorbers having absorption in a UV-A region) the compounds of the invention are not decomposed easily when exposed to light because of a high remaining ratio in a film.

## Claims

**1.** A compound represented by the following formula (1):

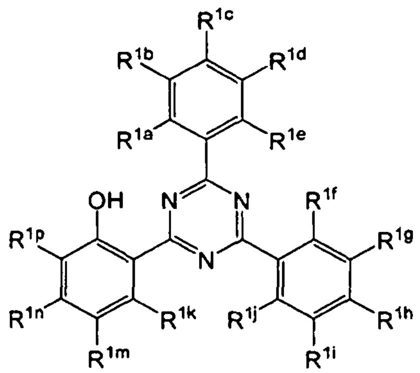

wherein $R^{1a}$, $R^{1b}$, $R^{1c}$, $R^{1d}$, and $R^{1e}$ each independently represents a hydrogen atom or a monovalent substituent other than OH with a proviso that at least one substituent has a positive σp value in Hammett equation and substituents may be coupled to each other to form a ring; $R^{1f}$, $R^{1g}$, $R^{1h}$, $R^{1i}$, and $R^{1j}$ each independently represents a hydrogen atom or a monovalent substituent other than OH with a proviso that substituents may be coupled to each other to form a ring; and $R^{1k}$, $R^{1m}$, $R^{1n}$, and $R^{1p}$ each independently represents a hydrogen atom or a monovalent substituent with a proviso that substituents may be coupled to each other to form a ring, and
wherein both $R^{1c}$ and $R^{1h}$ are substituents having a positive σp value in the Hammett equation.

2. The compound according to claim 1, wherein
the monovalent substituent is a halogen atom, a substituted or unsubstituted alkyl group having 1 to 20 carbon atoms, a cyano group, a carboxyl group, a substituted or unsubstituted alkoxycarbonyl group, a substituted or unsubstituted carbamoyl group, a substituted or unsubstituted alkylcarbonyl group, a nitro group, a substituted or unsubstituted amino group, a hydroxyl group, an alkoxy group having 1 to 20 carbon atoms, a substituted or unsubstituted aryloxy group, a substituted or unsubstituted sulfamoyl group, a thiocyanate group, or a substituted or unsubstituted alkylsulfonyl group, and
when the monovalent substituent is further substituted by a substituent, the substituted is a halogen atom, an alkyl group having 1 to 20 carbon atoms, a cyano group, a carboxyl group, an alkoxycarbonyl group, a carbamoyl group, an alkylcarbonyl group, a nitro group, an amino group, a hydroxyl group, an alkoxy group having 1 to 20 carbon atoms, an aryloxy group, a sulfamoyl group, a thiocyanate group, or an alkylsulfonyl group.

3. The compound according to Claim 1 or 2, wherein the σp value in Hammett equation falls within a range of from 0.1 to 1.2.

4. The compound according to any one of Claims 1 to 3, wherein the substituent having a positive σp value in Hammett equation is a group selected from $COOR^r$, $CONR^s_2$, CN, $CF_3$, halogen atoms, $NO_2$, $SO_2R^t$, $SO_3M$, wherein $R^r$, $R^s$, and $R^t$ each independently represents a hydrogen atom or a monovalent substituent and M represents a hydrogen atom or an alkali metal.

5. The compound according to any one of Claims 1 to 4, wherein the substituent having a positive σp value in Hammett equation is $COOR^r$, wherein $R^r$ represents a hydrogen atom or a monovalent substituent.

6. The compound according to any one of Claims 1 to 4, wherein $R^{1c}$ represents CN.

7. The compound according to any one of Claims 1 to 6, wherein $R^{1n}$ represents $OR^U$, wherein $R^U$ represents a hydrogen atom or a monovalent substituent.

8. The compound according to Claim 7, wherein $R^U$ represents an alkyl group having 1 to 20 carbon atoms.

9. The compound according to any one of Claims 1 to 8, which has pKa within a range of from -5.0 to -7.0.

10. An ultraviolet absorber comprising a compound as claimed in any one of Claims 1 to 9.

11. A resin composition comprising a compound as claimed in any one of Claims 1 to 9.

**Patentansprüche**

1. Verbindung, dargestellt durch die folgende Formel (1):

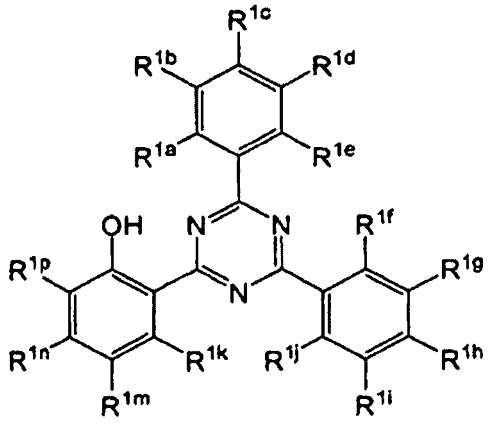

worin $R^{1a}$, $R^{1b}$, $R^{1c}$, $R^{1d}$ und $R^{1e}$ jeweils unabhängig voneinander ein Wasserstoffatom oder einen von OH verschiedenen monovalenten Substituenten darstellen, mit der Maßgabe, dass mindestens ein Substituent einen positiven σp-Wert in der Hammett-Gleichung aufweist und Substituenten zur Bildung eines Rings miteinander verbunden sein können, $R^{1f}$, $R^{1g}$, $R^{1h}$, $R^{1i}$ und $R^{1j}$ jeweils unabhängig voneinander ein Wasserstoffatom oder einen von OH verschiedenen monovalenten Substituenten darstellen, mit der Maßgabe, dass Substituenten zur Bildung eines Rings miteinander verbunden sein können, und $R^{1k}$, $R^{1m}$, $R^{1n}$ und $R^{1p}$ jeweils unabhängig voneinander ein Wasserstoffatom oder einen monovalenten Substituenten darstellen, mit der Maßgabe, dass Substituenten zur Bildung eines Rings miteinander verbunden sein können, und
worin sowohl $R^{1c}$ als auch $R^{1h}$ Substituenten sind, die einen positiven σp-Wert in der Hammett-Gleichung aufweisen.

2. Verbindung gemäß Anspruch 1, worin der monovalente Substituent ein Halogenatom, eine substituierte oder unsubstituierte Alkylgruppe mit 1 bis 20 Kohlenstoffatomen, eine Cyanogruppe, eine Carboxylgruppe, eine substituierte oder unsubstituierte Alkoxycarbonylgruppe, eine substituierte oder unsubstituierte Carbamoylgruppe, eine substituierte oder unsubstituierte Alkylcarbonylgruppe, eine Nitrogruppe, eine substituierte oder unsubstituierte Aminogruppe, eine Hydroxylgruppe, eine Alkoxgruppe mit 1 bis 20 Kohlenstoffatomen, eine substituierte oder unsubstituierte Aryloxygruppe, eine substituierte oder unsubstituierte Sulfamoylgruppe, eine Thiocyanatgruppe oder eine substituierte oder unsubstituierte Alkylsulfonylgruppe ist, und
wenn der monovalente Substituent weiterhin durch einen Substituenten substituiert ist, das Substituierte ein Halogenatom, eine Alkylgruppe mit 1 bis 20 Kohlenstoffatomen, eine Cyanogruppe, eine Carboxylgruppe, eine Alkoxycarbonylgruppe, eine Carbamoylgruppe, eine Alkylcarbonylgruppe, eine Nitrogruppe, eine Aminogruppe, eine Hydroxylgruppe, eine Alkoxygruppe mit 1 bis 20 Kohlenstoffatomen, eine Aryloxygruppe, eine Sulfamoylgruppe, eine Thiocyanatgruppe oder eine Alkylsulfonylgruppe ist.

3. Verbindung gemäß Anspruch 1 oder 2, worin der σp-Wert in der Hammett-Gleichung in einen Bereich von 0,1 bis 1,2 fällt.

4. Verbindung gemäß irgendeinem der Ansprüche 1 bis 3, worin der Substituent mit einem positiven σp-Wert in der Hammett-Gleichung eine Gruppe ausgewählt aus $COOR^r$, $CONR^s_2$, CN, $CF_3$, Halogenatomen, $NO_2$, $SO_2R^t$, $SO_3M$, worin $R^r$, $R^s$ und $R^t$ jeweils unabhängig voneinander ein Wasserstoffatom oder einen monovalenten Substituenten darstellen und M ein Wasserstoffatom oder ein Alkalimetall darstellt.

5. Verbindung gemäß irgendeinem der Ansprüche 1 bis 4, worin der Substituent mit dem positiven σp-Wert in der Hammett-Gleichung $COOR^r$ ist, worin $R^r$ ein Wasserstoffatom oder einen monovalenten Substituenten darstellt.

6. Verbindung gemäß irgendeinem der Ansprüche 1 bis 4, worin $R^{1c}$ CN darstellt.

7. Verbindung gemäß irgendeinem der Ansprüche 1 bis 6, worin $R^{1n}$ $OR^u$ darstellt, worin $R^u$ ein Wasserstoffatom oder einen monovalenten Substituenten darstellt.

8. Verbindung gemäß Anspruch 7, worin $R^u$ eine Alkylgruppe mit 1 bis 20 Kohlenstoffatomen darstellt.

9. Verbindung gemäß irgendeinem der Ansprüche 1 bis 8, die einen pKa-Wert innerhalb eines Bereichs von -5,0 bis -7,0 aufweist.

10. Ultraviolettabsorber, umfassend eine Verbindung gemäß irgendeinem der Ansprüche 1 bis 9.

11. Harzzusammensetzung, umfassend eine Verbindung gemäß irgendeinem der Ansprüche 1 bis 9.

**Revendications**

1. Composé représenté par la formule (1) suivante :

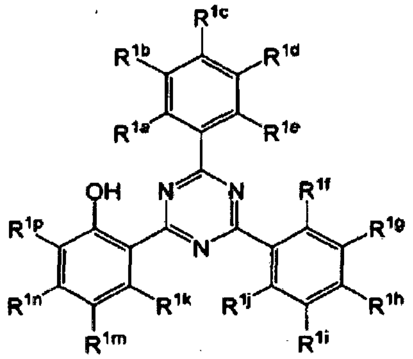

dans laquelle $R^{1a}$, $R^{1b}$, $R^{1c}$, $R^{1d}$, et $R^{1e}$ représentent chacun indépendamment un atome d'hydrogène ou un substituant monovalent différent de OH à condition qu'au moins un substituant présente une valeur σp positive dans une équation de Hammett et des substituants peuvent être couplés les uns aux autres pour former un cycle ; $R^{1f}$, $R^{19}$, $R^{1h}$, $R^{1i}$, et $R^{1j}$ représentent chacun indépendamment un atome d'hydrogène ou un substituant monovalent différent de OH à condition que des substituants peuvent être couplés les uns aux autres pour former un cycle ; et $R^{1k}$, $R^{1m}$, $R^{1n}$ et $R^{1p}$ représentent chacun indépendamment un atome d'hydrogène ou un substituant monovalent à condition que des substituants peuvent être couplés les uns aux autres pour former un cycle, et
dans laquelle à la fois $R^{1c}$ et $R^{1h}$ sont des substituants présentant une valeur σp positive dans l'équation d'Hammett.

2. Composé selon la revendication 1, dans lequel
le substituant monovalent est un atome d'halogène, un groupe alkyle substitué ou non substitué ayant de 1 à 20 atomes de carbone, un groupe cyano, un groupe carboxyle, un groupe alcoxycarbonyle substitué ou non substitué, un groupe carbamoyle substitué ou non substitué, un groupe alkylcarbonyle substitué ou non substitué, un groupe nitro, un groupe amino substitué ou non substitué, un groupe hydroxyle, un groupe alcoxy ayant de 1 à 20 atomes de carbone, un groupe aryloxy substitué ou non substitué, un groupe sulfamoyle substitué ou non substitué, un groupe thiocyanate, ou un groupe alkylsulfonyle substitué ou non substitué, et
lorsque le substituant monovalent est de plus substitué par un substituant, le substitué est un atome d'halogène, un groupe alkyle ayant de 1 à 20 atomes de carbone, un groupe cyano, un groupe carboxyle, un groupe alcoxycarbonyle, un groupe carbamoyle, un groupe alkylcarbonyle, un groupe nitro, un groupe amino, un groupe hydroxyle, un groupe alcoxy ayant de 1 à 20 atomes de carbone, un groupe aryloxy, un groupe sulfamoyle, un groupe thiocyanate, ou un groupe alkylsulfonyle.

3. Composé selon la revendication 1 ou 2, dans lequel la valeur σp dans l'équation d'Hammett se trouve dans un intervalle de 0,1 à 1,2.

4. Composé selon l'une quelconque des revendications 1 à 3, dans lequel le substituant présentant une valeur σp positive dans l'équation d'Hammett est un groupe choisi parmi $COOR^r$, $CONR^s_2$, CN, $CF_3$, des atomes d'halogène, $NO_2$, $SO_2R^t$, $SO_3M$, où $R^r$, $R^s$, et $R^t$ représentent chacun indépendamment un atome d'hydrogène ou un substituant monovalent et M représente un atome d'hydrogène ou un métal alcalin.

5. Composé selon l'une quelconque des revendications 1 à 4, dans lequel le substituant présentant une valeur σp

positive dans l'équation d'Hammett est $COOR^r$, où $R^r$ représente un atome d'hydrogène ou un substituant monovalent.

6. Composé selon l'une quelconque des revendications 1 à 4, dans lequel $R^{1c}$ représente CN.

7. Composé selon l'une quelconque des revendications 1 à 6, dans lequel $R^{1n}$ représente $OR^U$, où $R^U$ représente un atome d'hydrogène ou un substituant monovalent.

8. Composé selon la revendication 7, dans lequel $R^U$ représente un groupe alkyle ayant de 1 à 20 atomes de carbone.

9. Composé selon l'une quelconque des revendications 1 à 8, lequel présente un pKa dans l'intervalle de -5,0 à -7,0.

10. Absorbeur d'ultraviolets comprenant un composé selon l'une quelconque des revendications 1 à 9.

11. Composition de résine comprenant un composé selon l'une quelconque des revendications 1 à 9.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5339033 A
- JP 5345639 A
- JP 6056466 A
- JP 2002524452 T
- EP 1213283 A
- JP 7188190 A
- JP 11315072 A
- JP 2001220385 A
- JP 55129136 A
- JP 58209735 A
- JP 63264748 A
- JP 4191851 A
- JP 8272058 A
- GB 2016017 A
- JP 2008273950 A
- JP 2002053824 A


### Non-patent literature cited in the description


- **HANSCH, C. ; LEO, A. ; TAFT, R.W.** *Chem. Rev.,* 1991, vol. 91, 165-195
- *Dyestuffs & chemicals,* 1995, vol. 40 (12), 325-339
- The fourth series of Experimental Chemistry 7: Spectroscopy II. Maruzen, 1992, 180-186
- The fourth series of Experimental Chemistry 3: Basic Operation III. Maruzen, 1991, 154
- **SUMIO TOKITA.** Chemistry Seminar 9: Color Chemistry. Maruzen, 1982, 154-155
- New Series of Experimental Chemistry 9: Analytical Chemistry [II]. Maruzen, 1977, 244
- *Fine Chemical,* May 2004, 28-38
- Development of high-molecular functional additives. Toray Research Center, 1999, 96-140
- Development of high molecular additive and environmental measures. CMC, 2003, 54-64